# EUROPÄISCHE PATENTSCHRIFT

(11) **EP 0 661 051 B1**
(45) Veröffentlichungstag und Bekanntmachung des Hinweises auf die Patenterteilung: **11.01.2006**
(21) Anmeldenummer: 94115833.9
(22) Anmeldetag: 07.10.1994
(51) Int. Cl.: A61K 31/385, A61P 9/12

(54) **Präparat zur Behandlung von Bluthochdruck.**
Medicament for the treatment of hypertension.
Médicament pour le traitement de l'hypertension.

(30) Priorität: 12.10.1993 AT 204593; 19.10.1993 AT 209493
(43) Veröffentlichungstag der Anmeldung: 05.07.1995
(73) Patentinhaber: VIATRIS GmbH & Co. KG, 61352 Bad Homburg (DE)
(72) Erfinder: Költringer, Peter, Dr., A-8041 Graz (AT)

(56) Entgegenhaltungen:
- EP-A- 0 326 034
- EP-A- 0 382 066
- WO-A-89/03837
- DE-A- 3 504 933
- DE-A- 4 002 706
- STELLENWERT VON ANTIOXIDANTIEN BEIM DIABETES MELLITUS (HERAUSGEBER F.A.GRIES/K. WESSEL, Oktober 1993 Seiten 139 - 154 P.RÖSEN 'Schutzt Vitamin E das Gefässsystem des Diabetikers?'
- STELLENWERT VON ANTIOXIDANTIEN BEIM DIABETES MELLITUS (HERAUSGEBER F.A.GRIES/K.WESSEL), Oktober 1993 Seiten 170 - 181 L.PACKER 'Die mögliche Bedeutung von Thioctsäure und Vitamin E bei der diabetischen Polyneuropathie'
- ARZNEIMITTELFORSCHUNG, Bd.43, Nr.4, April 1993 Seiten 425 - 432 H.ASSADNAZARI ET AL. 'Cardioprotective Efficiency of dihydrolipoic Acid in Working Rat Hearts during Hypoxia and Reoxygenation'
- CHEMICAL ABSTRACTS, vol. 82, no. 13, 31. März 1975, Columbus, Ohio, US; abstract no. 80482q, V.N.IVANOV 'Effect of lipoic acid on tissue respiration in rabbits with experimental atherosclerosis' & COR VASA, Bd.16, Nr.2, 1974 Seite 141-150
- CHEMICAL ABSTRACTS, vol. 88, no. 13, 27. März 1978, Columbus, Ohio, US; abstract no. 84192s, H.SPRINCE ET AL. 'L-Ascorbic acid in alcoholism and smoking: protection against acetaldehyde toxicity as an experimental model' & INT.J.VITAMIN.NUTR.RES.,BEIH., Bd.16, 1977 Seiten 185 - 217
- CHEMICAL ABSTRACTS, vol. 70, no. 23, 9. Juni 1969, Columbus, Ohio, US; abstract no. 105092e, V.G.IVKOV ET AL. 'Effect of alpha-lipoic (thioctic) acid on the content of cholesterol and lecithin of the blood of arteriosclerosis patients' & NAUCH.TR.ASPIR.ORDINATOROV, 1-I MOSK.MED.INST., 1967 Seiten 292 - 295
- CHEMICAL ABSTRACTS, vol. 67, no. 25, 18. Dezember 1967, Columbus, Ohio, US; abstract no. 115722n, I.N.YAKOVLEVA 'Effect of lipoic acid on lipid metabolism and on cell permeability in the middle-aged and senile' & VITAMINY PREDUPREZHDENII LECH. PREZHDEVREMENNOGO STARENIYA, INST. GERONTOL. AKAD. MED NAUK. SSSR, 1966 Seiten 196 - 203
- FREE RAD. RES. COMMS., Bd.17, Nr.3, 1992 Seiten 211 - 217 Y.J.SUZUKI ET AL. 'LIPOATE PREVENTS GLUCOSE-INDUCED PROTEIN MODIFICATIONS'
- FREE RAD. RES. COMMS., Bd.17, Nr.1, 1992 Seiten 49 - 58 E.SERBINOVA ET AL. 'THIOCTIC ACID PROTECTS AGAINST ISCHEMIA-REPERFUSION INJURY IN THE ISOLATED PERFUSED LANGENDORFF HEART'
- J.BIOSCI., Bd.9, Nr.1&2, September 1985 Seiten 117 - 127 V.M.GANDHI ET AL. 'Lipoic acid and diabetes II: Mode of action of lipoic acid'
- J.BIOSCI., Bd.6, Nr.1, März 1984 Seiten 37 - 46 C.V.NATRAJ ET AL. 'Lipoic acid and diabetes: Effect of dihydrolipoic acid administration in diabetic rats and rabbits'
- Z.GESAMTE INN.MED., Bd.48, Nr.5, Mai 1993 Seiten 223 - 232 W.KÄHLER ET AL. 'Diabetes mellitus - eine mit Freien Radikalen assoziierte Erkrankung'
- J.LAB.CLIN.MED., Bd.124, Nr.3, September 1994 Seiten 313-314 - 359-370 K.STONE ET AL./C.A.O'NEILL ET AL. 'Cigarettes: Then and now / Aldehyde-induced protein modifications in human plasma: Protection by glutathione and dihydrolipoic acid'
- DATABASE MEDLINE [Online] US NATIONAL LIBRARY OF MEDICINE (NLM), BETHESDA, MD, US 1977 Database accession no. 77257963
- DATABASE MEDLINE [Online] US NATIONAL LIBRARY OF MEDICINE (NLM), BETHESDA, MD, US 1999 Database accession no. 99383609
- DATABASE MEDLINE [Online] US NATIONAL LIBRARY OF MEDICINE (NLM), BETHESDA, MD, US 1999 Database accession no. 20132404
- FREE RAD. RES., Bd. 20, Nr. 2, 1994, Seiten 119-133,
- ARZNEIMITTELFORSCHUNG, Bd. 43, Nr. 3, 1993, Seiten 425-432,
- FREE READ. RES. COMMS, Bd. 17, Nr. 1, 1992, Seiten 49-59,

## Beschreibung

α-Liponsäure (= Thioctsäure) ist chemisch 1,2-Dithia-cyclopentan-3-valeriansäure. Die Herstellung der freien R-Thioctsäure ist beispielsweise in der DE-OS 41 37 773 beschrieben.

Die Erfindung bezieht sich auf R,S-Thioctsäure in reduzierter oder oxidierter Form, ihre isolierten Enantiomere (R-Thioctsäure und S-Thioctsäure) und Metaboliten in Form der freien Säure oder als Salze. Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen α-Ketosäuren. Thioctsäure wird seit längerer Zeit bei verschiedenen Erkrankungen eingesetzt, so unter anderem bei Lebererkrankungen, bei Leberschädigungen durch Pilzvergiftung sowie bei diabetischer und alkoholischer Polyneuropathie, einer mit Stoffwechselerkrankungen einhergehenden Veränderung peripherer Nerven.

Chemie und Biochemie der α-Liponsäure werden in dem Artikel von Schmidt, Grafen und Goedde, Angw. Chem. 77 (1965), S. 900-911 und von Reed, Vitamines, Hormones 20 (1962) S. 1-38 diskutiert.
Die Biosynthese von Derivaten der α-Liponsäure wird von Gunsalus, Barton und Gruber in J. Amer. Chem. Soc. 78 (1956) S. 1763-1766 diskutiert.
Im Stand der Technik ist bereits die Verwendung von Dihydroliponsäure als Arzneimittel zur Behandlung von Schmerz- und Entzündungserkrankungen aus DE 40 02 706 bzw. EP 0 382 066 sowie der Liponsäure in Kombination mit Vitaminen als Arzneimittel für diverse Indikationen aus der EP 0 572 922 bekannt. Ebenfalls beschrieben wurde die Verwendung eines Kombinationspräparates aus Thioctsäure und Ginkgo biloba Extrakt zur Behandlung von Nervenzell- und Nervenfasererkrankungen (EP 0 326 034).

Die positive Wirkung von Dihydroliponsäure auf die Durchblutung nach Hypoxie-Reperfusion konnte am Rattenherz gezeigt werden (Arzneim.-Forsch. 43 (I), Nr. 4, S. 425-432 (1993)). Ebenso gibt es Hinweise für einen therapeutischen Effekt der Liponsäure bei der Behandlung von ischämischen Reperfusionsschäden im Herz (Free Rad. Res. Comms. 17 No 1, S. 49-58 (1992)).

Die vorliegende Erfindung betrifft eine Substanz, welche in einer neuen Anwendung gute Effekte zur Behandlung, oder zur Verlangsamung der Progredienz von Bluthochdruck bringt, beispielsweise bei:
a) Rauchern
b) Zuckerkranken
c) Patienten mit Fettstoffwechselstörungen

Weiterhin eignet sich das erfindungsgemäße Präparat für den prophylaktischen Einsatz bei den obengenannten Patienten, um zu verhindern, daß es überhaupt zu den Veränderungen kommt, die klinisch bis zur Ausbildung des Bluthochdruck führen können.

### Pharmakologisches Beispiel 1:

Männliche Wistar-Ratten mit einem durchschnittlichen Ausgangsgewicht um 120 g wurden verwendet. Die Kontrolltiere erhielten standardisiertes Rattenfutter. 30 Tiere (je 10 pro Gruppe) bekamen 10 g/Tier/Tag von einer speziellen cardiovasopathogenen Diät (Zusammensetzung s. unten). Wasser stand den Tieren ad libitum zur Verfügung. Eine Gruppe (10 Tiere) wurde täglich mit 0,5 ml/100 g 0,9 % NaCl-Lösung i.p. behandelt. Die Tiere der zwei weiteren Gruppen (jeweils 10 Ratten) wurden täglich mit Thioctsäure (15 bzw. 30 mg/kg) i.p. behandelt. Der Blutdruck wurde in wöchentlichen Abständen unblutig mit Hilfe eines geeigneten Blutdruckmeßgerätes gemessen.

| Gruppe | Blutdruckanstieg nach 7 Wochen (mm Hg) |
|---|---|
| Kontrolle | 3,5 |
| pathogene Diät | 31,4 |
| 15 mg/kg | 14 |
| 30 mg/kg | 14,5 |

Der Versuch wurde gemäß der Methode "Wirkung des Magnesium-Orotats und der Orotsäure auf die tierexperimentell ausgelöste Blutdruckerhöhung und kardiopathogene Veränderungen im Herzmuskel" von 1. Szelenyi, J. Sos und J. Rigo (Deutsches Medizinisches Joumal, 21. Jahrgang, Heft 22 - Berlin 20. November 1970) durchgeführt.

Viele der oben angeführten Erkrankungen beginnen schleichend und ohne klare Symptome: Unabhängig von der Grunderkrankung treten häufig Beschwerden an den Beinen auf. Die Patienten müssen oft stehenbleiben, da sie starke Schmerzen haben. Daneben gibt es aber auch Veränderungen am Herzen, welche von der Angina pectoris bis hin zum Infarkt führen können und auch Veränderungen im Gehirn, welche bei leichten Fällen vorübergehende Mangeldurchblutungen verursachen oder aber sich in Form von Lähmungen, oder anderen neurologischen Symptomen auf Dauer auswirken können.

Der Wirkmechanismus vom Auftreten von Gefäßveränderungen, welche in der Folge die Durchblutung beeinträchtigen, läuft zu einem wesentlichen Teil über die Blutplättchen, welche in der Medizin als Thrombozyten bezeichnet werden. Die Blutplättchen haben an sich eine physiologische Funktion zur Reparation von Gewebeschäden, in dem sie sich dort anlagern, wo Kollagen vorhanden ist. Dies bedeutet für das Gefäßsystem, daß an Stellen, wo die innere Schicht defekt ist, Kollagen angeboten wird und sich gesunde Thrombozyten anlagern, um eine Auskleidung und eine Regeneration zu ermöglichen. Bei bestimmten Erkrankungen, vor allem aber auch bei Rauchern, ist der einzelne Thrombozyt überempfindlich, das heißt, er reagiert bereits auf kleinste Kollagenmengen mit Anlagem an der Gefäßwand. Das hat zu Folge, daß sich nun Thrombozyten auf einer praktisch gesunden Gefäßwand auflagern und so das Gefäß einengen. Daß dies in der Folge zu einer Abnahme der Strömung führen muß, ist zwangsläufig. Ein protektiver Effekt kommt hier, wie schon in "Stellenwert von Antioxidantien beim Diabetes mellitus" (Hrsg. F.A. Gries/K. Wessel, Oktober 1993, S. 139-154) beschrieben, dem Antioxidans Vitamin E zu. Dies kann insbesondere beim Diabetes mellitus die Thrombozytenaktivierung und Thrombozytenaggregation hemmen.

Die vorliegende Erfindung hat es sich zum Ziel gesetzt, hier Abhilfe zu schaffen. Zur Lösung dieser Aufgabe sieht die Erfindung die Alpha-Liponsäure (Thioctsäure) als Substanz vor, welche speziell die überempfindlichen Thrombozyten in ihrer Funktion "bremst" und ihre physiologischen Funktionen normalisiert. im Vergleich zu anderen Substanzen, welche ebenfalls auf die Thrombozytenfunktion wirken, treten bei Alpha-Liponsäure kaum Nebenwirkungen auf.
Alpha-Liponsäure, auch Thioctsäure genannt, ist im Handel in Tabletten und Ampullenform zugelassen, wobei die Dosierungen bei Tabletten um 200 mg liegen, bei Ampullen bewegt sich die Dosierung in der Größenordnung von 10 mg bis 50 mg/ml. Als Hauptindikationsgebiete gelten die diabetische Polyneuropathie sowie in manchen Ländern auch bestimmte Lebererkrankungen.

Bei Rauchern zeigte sich, daß die Alpha-Liponsäure prophylaktisch gegeben den an sich für Raucher typischen Thrombozytenfunktions-steigernden Effekt weitgehend beseitigt. Darüber hinaus fand sich auch bei zahlreichen Rauchern unter Alpha-Liponsäure eine deutlich bessere Hautdurchblutung. Bei Patienten mit Zuckerstoffwechselstörungen (Diabetes mellitus) hat sich gezeigt, daß im Frühstadium ebenfalls längere Zeit weitgehend normale Thrombozytenfunktionen auftreten, was ungewöhnlich erscheint und für eine verbesserte Durchblutung spricht.

Aufgrund der bisherigen klinischen Erfahrungen gibt es Indizien dafür, daß der Erfindungsgegenstand die Zunahme von Durchblutungsstörungen und Ausbildung vom Bluthochdruck bremst und andererseits, wenn z.B. prophylaktisch bei Diabetikern gegeben, daß Auftreten dieser Störungen zumindest massiv verzögert.

Die Erfindung wird nachfolgend anhand von Beispielen näher erläutert.

### Beispiel 2

Bei Rauchern mit intravenöser Gabe von 1 Ampulle Alpha-Liponsäure über 7 Tage zeigte sich, daß sich die Thrombozytenfunktionswerte auf Kollagen unauffällig waren, wobei bei einer altersgleichen Rauchergruppe ohne Alpha-Liponsäure deutlich höhere Werte registriert wurden. Zusätzlich durchgeführte Durchblutungsmessungen an der Haut der Beine zeigte in der Alpha-Liponsäure-Gruppe deutlich höhere werte als ohne Alpha-Liponsäure, was für eine Durchblutungsverbesserung in der Mikrozirkulation spricht.

### Beispiel 3

Bei einer freiwilligen Versuchsperson (Nichtraucher) wurden die Werte der Thrombozytenfunktion bestimmt, welche unauffällig waren. Nach dem Rauchen von 1 Zigarette fanden sich deutlich höhere Werte. Das gleiche Experiment wurde am nächsten Tag wiederholt, wobei zuvor eine Dosis von 400 mg Alpha-Liponsäure in Tablettenform eingenommen wurde. Es kam zu keinem Anstieg der Werte.

### Beispiel 4

An Patienten mit Zuckerkrankheit seit mehreren Jahren wurde über 1 Woche Alpha-Liponsäure verabreicht. Diese Gruppe zeigte unauffällige Thrombozytenwerte. Eine weitgehend identische Kontrollgruppe wies hingegen deutlich höhere Werte auf.

## Patentansprüche

1. Verwendung von alpha-Liponsäure (R,S-Thioctsäure in reduzierter oder oxidierter Form, ihre isolierten Enantiomere, in Form der freien Säure, als Salze) zur Herstellung eines Präparats zur Behandlung, zur Verhinderung oder zur Verlangsamung der Progredienz von Bluthochdruck durch Einsatz bei Rauchern, Zuckerkranken und Patienten mit Fettstoffwechselstörungen.

2. Verwendung nach Anspruch 1, wobei die Alpha-Liponsäure bei Gefäßerkrankungen, welche durch Diabetes Typ I und Typ II, oder seiner Frühform (Insulinresistenz) bedingt sind, eingesetzt wird.

3. Verwendung nach einem der vorstehenden Ansprüche, wobei eine Mindestdosis der Liponsäure von 50 mg intravenös oder 20 mg oral verabreicht wird.

4. Verwendung nach einem der Ansprüche 1 bis 3, wobei die Alpha-Liponsäure über einen längeren Zeitraum, zumindest jedoch über 7 Tage angewendet wird.

## Claims

1. Use of alpha-lipoic acid (R,S-thioctic acid in reduced or oxidized form, its isolated enantiomers, in the form of the free acid, as salts) for the manufacture of a product for the treatment, for the prevention or for slowing the progression of high blood pressure through use in smokers, diabetics and patients with disorders of lipid metabolism.

2. Use according to Claim 1, where the alpha-lipoic acid is employed for vascular disorders which are caused by diabetes of type I and type II, or its early form (insulin resistance).

3. Use according to either of the preceding claims, where a minimum dose of lipoic acid of 50 mg intravenous or 20 mg oral is administered.

4. Use according to any of Claims 1 to 3, where the alpha-lipoic acid is used over a prolonged period, but for at least 7 days.

## Revendications

1. Utilisation de l'acide α-lipoïque (acide R,S-thioctique sous forme réduite ou oxydée, ses énantiomères isolés, sous forme de l'acide libre, sous forme de sels) pour la préparation d'une préparation destinée au traitement, à la prévention ou au ralentissement de la progrédience de l'hypertension artérielle par l'utilisation chez les fumeurs, les diabétiques et les patients souffrant de troubles du métabolisme des graisses.

2. Utilisation selon la revendication 1, l'acide α-lipoïque étant utilisé dans le cas de maladies vasculaires provoquées par le diabète de type I et de type II ou sa forme précoce (résistance à l'insuline).

3. Utilisation selon l'une quelconque des revendications précédentes, en administrant une dose minimale de l'acide lipoïque de 50 mg par voie intraveineuse ou de 20 mg par voie orale.

4. Utilisation selon l'une quelconque des revendications 1 à 3, l'acide α-lipoïque étant utilisé sur une longue période, toutefois au moins pendant 7 jours.
